# EUROPÄISCHE PATENTSCHRIFT

(11) **EP 1 121 426 B1**
(45) Veröffentlichungstag und Bekanntmachung des Hinweises auf die Patenterteilung: **14.04.2004**
(21) Anmeldenummer: 99936433.4
(22) Anmeldetag: 02.06.1999
(51) Int. Cl.: C12N 15/12, A61K 48/00

(54) **VERFAHREN ZUR AUSLÖSUNG VON APOPTOSE IN ZELLEN**
METHOD FOR TRIGGERING APOPTOSIS IN CELLS
PROCEDE POUR DECLENCHER L'APOPTOSE DANS DES CELLULES

(30) Priorität: 03.06.1998 DE 19824811
(43) Veröffentlichungstag der Anmeldung: 08.08.2001
(73) Patentinhaber: Deutsches Krebsforschungszentrum Stiftung des öffentlichen Rechts, 69120 Heidelberg (DE); Nehls, Peter, 80339 München (DE)
(72) Erfinder: ROTHBARTH, Karsten, D-69493 Hirschberg (DE); STAMMER, Hermann, D-69221 Dossenheim (DE); WERNER, Dieter, D-69118 Heidelberg (DE); NEHLS, Peter, D-80339 München (DE)
(74) Vertreter: Schüssler, Andrea, Dr.
(86) Internationale Anmeldenummer: PCT/DE1999/001684
(87) Internationale Veröffentlichungsnummer: WO 1999/063071

(56) Entgegenhaltungen:
- NEHLS ET AL: "cDNA cloning, recombinant expression and characterization of polypetides with exceptional DNA affinity" NUCLEIC ACIDS RESEARCH, Bd. 26, Nr. 5, 1. März 1998 (1998-03-01), Seiten 1160-1166, XP002126990 in der Anmeldung erwähnt
- YAVUZER ET AL: "DNA end-independent activation of DNA-PK mediated via association with the DNA-binding protein C1D" GENES AND DEVELOPMENT, Bd. 12, 15. Juli 1998 (1998-07-15), Seiten 2188-2199, XP002126991
- DATABASE CHEMICAL ABSTRACTS [Online] AN=130:106573, HAATAJA ET AL: "Identification of a novel Rac3-interacting protein C1D" XP002126992 & INT. J. MOL. MED., Bd. 1, Nr. 4, 1998, Seiten 665-670,

## Beschreibung

Die vorliegende Erfindung betrifft ein Verfahren zur Auslösung von Apoptose in Zellen, insbesondere Tumorzellen.

Apoptose ist der programmierte Zelltod. Dieser unterliegt einer genauen Regulation, wobei Apoptose induziert bzw. inhibiert werden kann.

Die Induktion von Apoptose kann bekanntermaßen über eine Reihe von sog. Todesrezeptoren, d.h. Rezeptoren, die eine "Death Domain" (DD) enthalten, wie CD95, TNF-RI, DR3, DR4 oder DR5, erfolgen, die nach Bindung ihrer Liganden Apoptose-Signalwege induzieren. Beispielsweise interagiert nach Bindung des CD95-Liganden der CD95-Rezeptor mit dem Adapterprotein FADD/MORT1, wodurch das "Recruitment" und die Aktivierung der Protease FLICE/Caspase-8, am DISC "Death Inducing Signalling Complex" induziert werden. FADD und FLICE enthalten jeweils "Death Effector Domains" (DED). Die Induktion der Apoptose über diese Apoptose-Signalwege ist von außen beispielsweise durch die Gabe von Zellgiften (cytotoxischen Substanzen), Bestrahlung, Viren, Entzug von Wachstumsfaktoren oder mechanische Zellverletzung möglich. Diese Möglichkeiten der Apoptose-Induktion sind allerdings von bestimmten Nachteilen begleitet. So führt die Gabe von Zellgiften, wie Zytostatika, oder die Bestrahlung bei Krebszellen zur Resistenzentwicklung und darüberhinaus zu einer Schädigung normaler Zellen, bei denen eigentlich keine Apoptose ausgelöst werden sollte.

So besteht die Aufgabe der vorliegenden Erfindung darin, ein Verfahren bereitzustellen, mit der Apoptose, z.B. zur Bekämpfung von malignem Wachstum, unter Reduktion der oben beschriebenen Nebenwirkungen ausgelöst werden kann.

Erfindungsgemäß wird dies durch die Gegenstände in den Patentansprüchen erreicht.

Die vorliegende Erfindung beruht auf den Erkenntnissen der Erfinder, daß in Tieren, besonders Säugetieren ganz besonders dem Menschen, ein Protein vorliegt, das sich zur Induktion von Apoptose eignet. Ein solches Protein weist eine Größe von ca. 16 kD auf und wurde bisher als DNA-bindendes Protein charakterisiert (Mehls et al., Nucleic Acids Research, 26, S. 1160-1166 (1998).

Von den Erfindern wurde erkannt, daß das zur Induktion von Apoptose geeignete Protein (nachstehend mit C1D bezeichnet) in jeder Zelle, auch in Tumorzellen vorhanden ist, und dort in einer vom Organismus vorgegebenen Menge exprimiert wird. Kommt es zu einer Überexpression des C1D-Genprodukts, wird in den überexprimierenden Zellen Apoptose ausgelöst. Aber gerade in Tumorzellen ist eine durch Überexpression erzielte Apoptose wünschenswert. Diese Überexpression kann per se die Tumorzellen abtöten. Weiter könnte sie die durch die übliche Tumorbehandlung, wie Chemotherapie oder Bestrahlung, bewirkte Apoptose verstärken. Außerdem könnte in Tumorzellen Apoptose bewirkt werden, bei denen sich durch übliche Behandlungswege schon eine Resistenz entwickelt hat. Von den Erfindern wurde jetzt herausgefunden, daß die Auslösung von Apoptose dadurch bewirkt werden kann, daß das C1D-Gen zur Überexpression gebracht wird, d.h. die Konzentration der zellulären C1D-Genprodukts erhöht wird. Dies kann beispielsweise dadurch geschehen, daß die Zellen mit Expressionskonstrukten transfiziert werden, die das C1D-Gen exprimieren oder das endogene C1D-Gen zur Überexpression stimuliert wird.

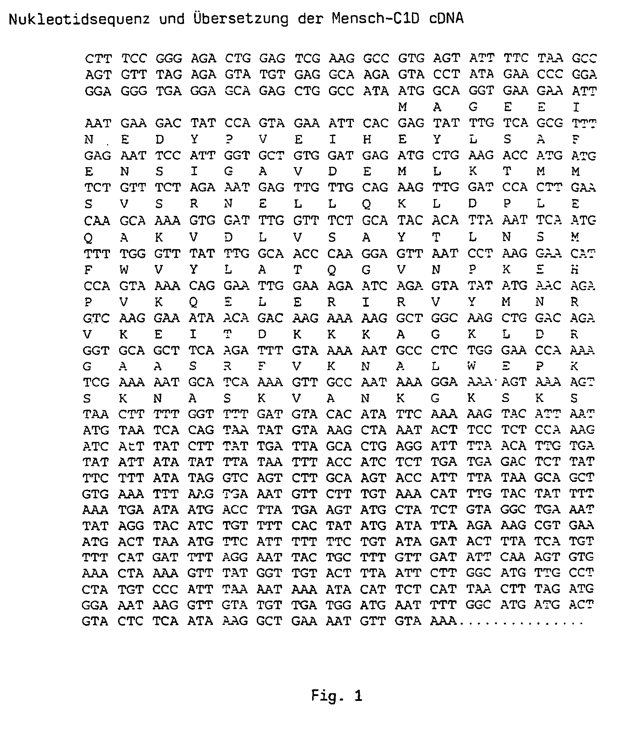

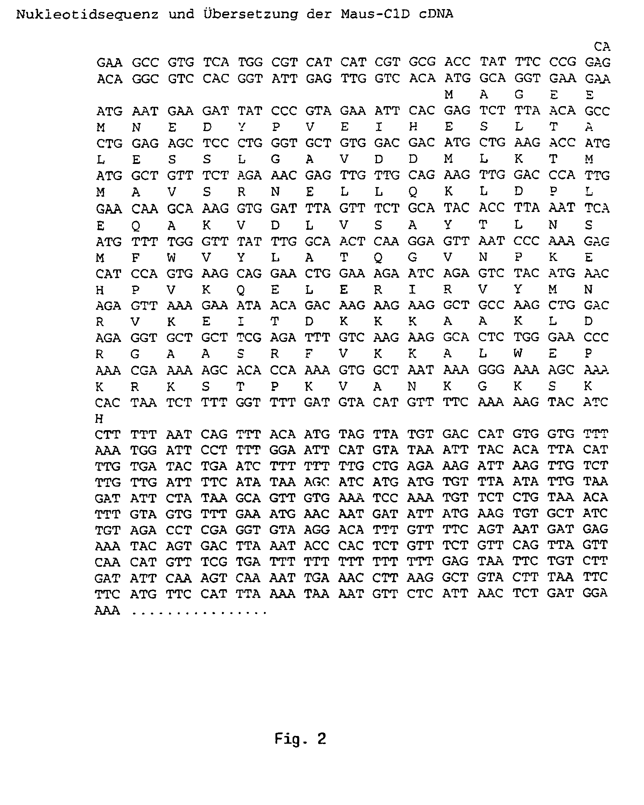

Das C1D-Genprodukt umfaßt die Sequenz von Fig. 1 bzw. 2 oder eine hiervon durch eine oder mehrere Aminosäuren unterschiedliche Aminosäuresequenz. Der Ausdruck "eine durch eine oder mehrere Aminosäuren unterschiedliche Aminosäuresequenz" umfaßt jegliche für ein C1D-(verwandtes) Protein kodierende Aminosäuresequenz, deren DNA-Sequenz mit der DNA von Fig. 1 bzw. 2 hybridisiert. Bezüglich des Begriffs "hybridisiert" wird auf die nachstehenden Ausführungen verwiesen.

Für die Ausführung des erfindungsgemäßen Verfahrens ist insbesondere eine für C1D kodierende Nukleinsäure in Form einer DNA, insbesondere cDNA, geeignet. Bevorzugt ist eine DNA, die folgendes umfaßt:
(a) Die DNA von Fig. 1 bzw. 2 oder eine hiervon durch ein oder mehrere Basenpaare unterschiedliche DNA, wobei letztere DNA mit der DNA von Fig. 1 oder 2 hybridisiert, oder
(b) eine mit der DNA von (a) über den degenerierten genetischen Code verwandte DNA.

Die Sequenzdaten der C1D cDNAs gemäß Fig. 1 bzw. 2 sind in der Genbank unter den folgenden Accessionnummern verfügbar:
Maus cDNA: X95591;
Mensch cDNA: X95592

Der Ausdruck "eine durch ein oder mehrere Basenpaare unterschiedliche DNA" umfaßt jegliche für ein C1D-(verwandtes) Protein kodierende DNA-Sequenz, die mit der DNA von Fig. 1 oder 2 hybridisiert. Die DNA kann sich von der DNA von Fig. 1 oder 2 durch Additionen, Deletionen, Substitutionen und/oder Inversionen von ein oder mehreren Basenpaaren oder andere im Stand der Technik bekannte Modifikationen, z.B. alternatives Splicing, unterscheiden. Erfindungsgemäß umfaßt der Begriff "DNA" auch Fragmente dieser DNA. Der Begriff "Fragment" soll einen Ausschnitt bzw. Segment des ursprünglichen Nucleinsäuremoleküls umfassen, wobei das durch dieses Fragment codierte Protein noch die Apoptose auslösenden Eigenschaften von C1D aufweist. Dazu zählen auch Allelvarianten. Verfahren zur Erzeugung der vorstehenden Änderungen in der Nucleinsäuresequenz sind dem Fachmann bekannt und in Standardwerken der Molekularbiologie beschrieben, beispielsweise in Sambrook et al., Molecular Cloning: A Laboratory Manual, 2. Ausgabe, Cold Spring Harbor Laboratory Press, Cold Spring Harbor NY (1989).

Der Fachmann ist auch in der Lage zu bestimmen, ob ein von einer so veränderten Nucleinsäuresequenz codiertes Protein noch über die biologische Aktivität der Apoptose-Induktion verfügt, z.B. durch Nachweis von Apoptose-typischem Zelltod gekennzeichnet durch z.B. Morphologie, multizentrische Chromatinkondensation, typische Membranveränderungen und endogene DNA-Degradation.

Der Ausdruck "DNA mit .... hybridisiert" weist auf eine DNA hin, die unter üblichen Bedingungen, insbesondere bei 20°C unter dem Schmelzpunkt der DNA, mit einer DNA von Fig. 1 bzw. 2 hybridisiert. Der Begriff "hybridisieren" bezieht sich dabei auf konventionelle Hybridisierungsbedingungen, vorzugsweise auf Hybridisierungsbedingungen, bei denen als Lösung 5xSSPE, 1% SDS, 1xDenhardts-Lösung verwendet wird und die Hybridisierungstemperaturen zwischen 35°C und 70°C, vorzugsweise bei 65°C liegen. Nach der Hybridisierung wird vorzugsweise zuerst mit 2xSSC, 1% SDS und danach mit 0,2xSSC bei Temperaturen zwischen 35°C und 70°C, vorzugsweise bei 65°C gewaschen (zur Definition von SSPE,SSC und Denhardts-Lösung siehe Sambrook et al.,supra). Besonders bevorzugt sind stringente Hybridisierungsbedingungen, wie sie beispielsweise in Sambrook et al ., supra, beschrieben sind.

Um das C1D-Genprodukt herzustellen, das zur Ausführung des erfindungsgemäßen Verfahrens geeignet ist, wird die für C1D kodierende DNA in einen Vektor bzw. Expressionsvektor inseriert, z.B. pBlueScript, pQE8, pUC- oder pBr322-Derivate. In einer bevorzugten Ausführungsform ist das erfindungsgemäße Nucleinsäuremolekül im Vektor mit regulatorischen Elementen funktionell verknüpft, die dessen Expression in eukaryotischen Wirtszellen erlauben. Solche Vektoren enthalten neben den regulatorischen Elementen, beispielsweise einem Promotor, typischerweise einen Replikationsursprung und spezifische Gene, die die phänotypische Selektion einer transformierten Wirtszelle erlauben. Zu den regulatorischen Elementen für die Expression in Eukaryonten zählen der CMV-, SV40-, RVS-40-Promotor, sowie CMV- oder SV40-Enhancer. Weitere Beispiele für geeignete Promotoren sind der Metallothionein I- und der Polyhedrin-Promotor.

In einer für gentherapeutische Zwecke bevorzugten Ausführungsform ist der die C1D-DNA enthaltende Vektor ein Virus, beispielsweise ein Adenovirus, Vaccinia-Virus oder Adeno-abhängige Parvoviren (AAV). Besonders bevorzugt sind Retroviren. Beispiele für geeignete Retroviren sind MoMuLV, HaMuSV, MuMTV, RSV oder GaLV. Für Zwecke der Gentherapie können die erfindungsgemäßen Nucleinsäuremoleküle auch in Form von kolloidalen Dispersionen zu den Zielzellen transportiert werden. Dazu zählen beispielsweise Lipososmen oder Lipoplexe (Mannino et al., Biotechniques 6 (1988), 682).

Allgemeine auf dem Fachgebiet bekannte Verfahren können zur Konstruktion von Expressions- und insbesondere Gentherapievektoren, die die oben genannten Nucleinsäuremoleküle und geeignete Kontrollsequenzen enthalten, verwendet werden. Zu diesen Verfahren zählen beispielsweise in vitro-Rekombinationstechniken, synthetische Verfahren, sowie in vivo-Rekombinationsverfahren, wie sie beispielsweise in Sambrook et al., supra, beschrieben sind. Der Fachmann weiß somit, in welcher Weise eine erfindungsgemäße DNA in einen Expressionsvektor inseriert werden muß. Ihm ist auch bekannt, daß diese DNA in Verbindung mit einer für ein anderes Protein bzw. Peptid kodierenden DNA inseriert werden kann, so daß die erfindungsgemäße DNA in Form eines Fusionsproteins exprimiert werden kann. Beispielsweise in Form eines Fusionsproteins, bei dem der andere Teil GFP (das grün fluoreszierende Protein von Aequorea Victoria) ist.

Für die Expression des C1D-Gens werden die oben genannten Expressionsvektoren in Wirtszellen eingeführt. Zu diesen Wirtszellen zählen Tierzellen, vorzugsweise Säugerzellen, sowohl in Kultur wie auch im lebenden Organismus. Bevorzugt sind die tierischen Zellen L, 3T3, FM3A, CHO, COS, Vero und HeLa. Verfahren zur Transformation dieser Wirtszellen, zur Erkennung erfolgter Transformation und Expression der erfindungsgemäßen Nucleinsäuremoleküle unter Verwendung der vorstehend beschriebenen Vektoren sind auf dem Fachgebiet bekannt.

Des weiteren kennt der Fachmann Bedingungen, transformierte bzw. transfizierte Zellen zu kultivieren. Auch sind ihm Verfahren bekannt, das durch die erfindungsgemäße DNA exprimierte Protein bzw. Fusionsprotein zu isolieren und zu reinigen.

Um das erfindungsgemäße Verfahren auszuführen, wird in einer bevorzugten Ausführungsform die C1D-DNA in einen Expressionsvektor, insbesondere einen Gentherapievekor, inseriert und in Zellen, bevorzugt Tumorzellen, eingeführt. Dort kommt es zur Expression von C1D-Protein, das zusätzlich zum zelleigenen Protein, zur Auslösung von Apoptose führt. Das Einbringen der Vektoren in die Zellen erfolgt unter den dem Fachmann bekannten Bedingungen. Hinsichtlich der in-vivo Gentherapie wird insbesondere auf "K.W. Culver, Gene Therapy, A Handbook for Physicans, Mary Ann Libert, Inc., New York , 1994" und "P.L. Chang, Sonatic Gene Therapy, CRC Press, London, 1995" verwiesen.

In einer weiteren bevorzugten Ausführungsform wird das zelleigene C1D-Gen zu einer vermehrten Expression stimuliert, z.B. durch exogene Stimulation des endogenen C1D-Promoters. Als Promoter bezeichnet man 5'-Nachbarsequenzen eines Gens, die als Startpunkte der RNA Polymerase II dienen, welche im Zusammenwirken mit Transkriptionsfaktoren die Expression des Gens bewirkt. Bei vielen Genen, wie auch bei C1D, ist dieser Prozeß durch exogene Faktoren induzierbar bzw. stimulierbar. Faktoren, die die spezifische Expression eines Gens bewirken sind sehr zahlreich und reichen von physikalischen Faktoren (wie Licht, Wärme, Kälte), über niedermolekulare anorganische Stoffe (wie Salze, Metallionen) und niedermolekulare organische Stoffe (Peptide, Nukleinsäurebausteiene, biogene Amine, Steroide) bis zu höhermolekularen Stoffen (Serum, Wachstumsfaktoren, Immun-Stimulantien). Die für das C1D-Gen spezifischen Stimulantien werden dadurch erkannt, daß 5'-Nachbarsequenzen, vorhanden auf z.B. den BAC (bacterial arteficial chromosome) Klonen mit einem Reportergen, z.B. CAT oder EGFP, kombiniert und hinsichtlich der Reportergen-Expression bzw. deren Stimulation durch exogene Faktoren, ggf. mit einem "high-throughput"-Verfahren, untersucht werden.

Somit stellt die vorliegende Erfindung erstmalig eine Möglichkeit bereit, Apoptose nicht über die üblichen Signalwege auszulösen, sondern durch Überexpression eines bestimmten Gens. Dies kann eine besondere Bedeutung bei vielen Erkrankungen haben, insbesondere Tumorerkrankungen. Insbesondere hat sich als vorteilhaft herausgestellt, daß Tumorzellen auf eine Überexpression von C1D sehr viel empfindlicher als normale Zellen reagieren. Für normale Zellen bestehen deshalb keinerlei Nebenwirkungen, während Tumorzellen den sicheren Zelltod erleiden.

### Kurze Beschreibung der Figuren:

- **Fig. 1**: zeigt die DNA- und Aminosäuresequenz von C1D aus Mensch
- **Fig. 2**: zeigt die DNA- und Aminosäuresequenz von C1D aus Maus
- **Fig. 3**: zeigt den zeitlichen Verlauf eines durch Überexpression von C1D ausgelösten Apoptoseprozesses in Zellen des Ehrlich Ascites Tumors (Fluoreszenzmikroskopie; Anregung: 480 nm, Emission: 520 nm)
- **Fig. 4**: zeigt Beispiele von morphologischen Besonderheiten im Verlauf eines durch Überexpression von C1D ausgelösten Apoptoseprozesses in Zellen des Ehrlich Ascites Tumors (Phasenkontrastaufnahmen)

Die vorliegende Erfindung wird durch die nachstehenden Beispiele erläutert.

### Beispiel 1: Induktion von Apoptose durch Expression des C1D-Gens

- pcDNA 3 - C1D-Expressionskonstrukte
   Die im Bluescript-Vektor (KS+, Fa. Stratagene) klonierte cDNA kodierend für menschliches bzw. murines C1D wurden durch PCR amplifiziert. Dabei wurden die folgenden Primer verwendet:
   für menschliche cDNA: 
Primer vorwärts:
5'-GGGGTACCATGGCAGGTGAAGAAATTAATGAAGACTAT
 
Primer rückwärts:
5'-GGGTCGACTTAACTTTTACTTTTTCCTTTATTGGCAAC
 (bewirkt Amplifikation der Nukleotidsequenz von Base 118 bis Base 540 gemäß Fig. 1)
   für Maus cDNA: 
Primer vorwärts:
5'-GGGGTACCATGGCAGGTGAAGAAATGAATGAAGATTAT
 
Primer rückwärts:
5'-GGGTCGACGTGTTTGCTTTTCCCTTTATTAGCCACTTT
 (bewirkt Amplifikation der Nukleotidsequenz von Base 78 bis Base 500 gemäß Fig. 2)

Mit diesen Primern wurde eine Kpn-Schnittstelle vor dem ATG-Startcodon und eine Sal I-Restriktionsstelle vor den Stop-Kodon eingeführt (sodaß das Stopkodon entfiel). Die PCR-Reaktion wurde mittels PCR Kit der Fa. Clontech (K1906-1) nach den Angaben des Herstellers unter Verwendung der Kitbestandteile in 50 µl Volumina durchgeführt:

| | |
|---|---|
| Wasser | 38,8 µl |
| 10x Puffer | 5 µl |
| Mg-Acetat | 2,2 µl |
| Primer-Vor | 1 µl (10 µM) |
| Primer-Rück | 1 µl (10 µM) |
| C1D-Templat | 5 µl (500 ng) |
| 50x dNTP | 1 µl |
| Kit-Polymerase | 1 µl |

### Cyclerprogramm:

| | |
|---|---|
| 1. Initiale Denaturierung | 94°C, 1 Min. |
| 2. Denaturierung | 94°C, 30 Sec. |
| 3. Annealing-Extension | 68°C, 3 Min. |
| 4. End-Extension | 68°C, 3 Min. |
| 5. Abkühlung | 4°C |
| Schritte 2/3 werden 35 mal durchgeführt | |

Nach Restriktionsverdau des Amplifikationsansatzes mit Kpn I/Sal I wurden die Fragmente zunächst im Bluescript-Vektor (Kpn I/Sal I - vorbehandelt) rekloniert. Nach Ausschneiden der Fragmente aus dem Bluescript-Vektor mit Kpn I/Not I konnten die Sequenzen im entsprechend vorbehandelten pcDNA 3-Vektor (Fa. InVitrogen) einkloniert werden.

### - pcDNA3-C1D-EGFP-Expressionskonstrukte

Die Fusion zwischen C1D und GFP (grün fluoreszierendes Protein von Aequorea Victoria) mit durchgehendem Leserahmen erfolgte auf der pBluescript-Ebene. Dazu wurden die oben beschriebenen pBluescript-(Kpn I)-C1D-(Sal I)-Plasmide durch Verdau mit Sal I/Hind III geöffnet.

Die für EGFP kodierende Sequenz (Fa. Clontech; EGFP bedeutet "enhanced green fluorescent protein" und ist eine von der Fa. Clontech hergestellte Mutante, die verbesserte Eigenschaften hinischtlich Excitation/Emission hat) wurde durch PCR amplifiziert. In dieser PCR wurden die folgenden Primer eingesetzt: 
Primer vorwärts:
5'-GGGTCGACATGGTGAGCAAGGGCGAGGAGCTGTTC
 
Primer rückwärts:
5'-CCAAGCTTTGGAATTCTAGAGTCGCGGCCGCTTTA
 um am 5-Ende eine Sal I-Stelle und am 3'-Ende (hier nach dem Stopcodon) eine Hind III-Stelle einzufügen. Die PCR wurde analog wie zuvor beschrieben durchgeführt.

Nach Verdau der PCR-Amplifikationsprodukte mit Sal I und Hind III konnten diese in die oben vorbereiteten Bluescript-(Kpn I)-C1D-(Sal I) ..... (Hind III)-Plasmide ligiert werden. Danach wurde die Fusionskassette (Kpn I)-C1D-EGFP-(NotI) durch entprechenden Verdau aus dem Bluescript-Vektor herausgeschnitten und in den entprechend vorbehandelten pcDNA 3-Vektor (Kpn I/Not I) rekloniert.

### - Transfektion der Vektoren in Tumorzellen

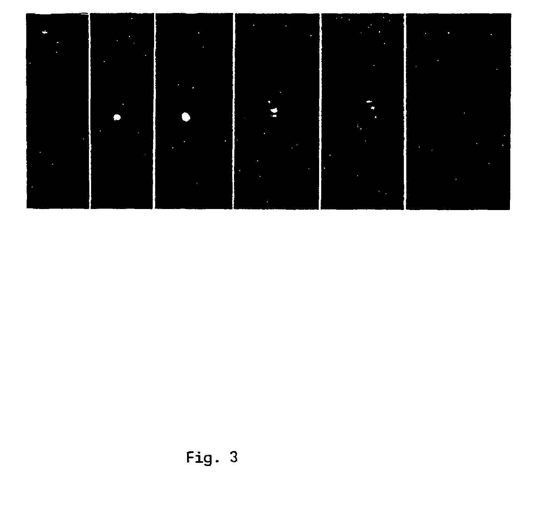

Die oben erhaltenen Expressionsvektoren wurden getrennt voneinander per Elektroporation (Potter et al., Proc. Natl. Acad. Sci. USA, 81, S. 7161-7165 (1984) bzw. Lipofektion (SuperFect Transfection Reagent Handbook, Fa. Qiagen, Hilden, 02/97, 1997) in Zellen des Ehrlich Ascites Tumors transfiziert. Transfizierte (lebende Zellen) wurden im Mikroskop (Fluoreszenzoptik) beobachtet und fotografiert (Fig. 3).

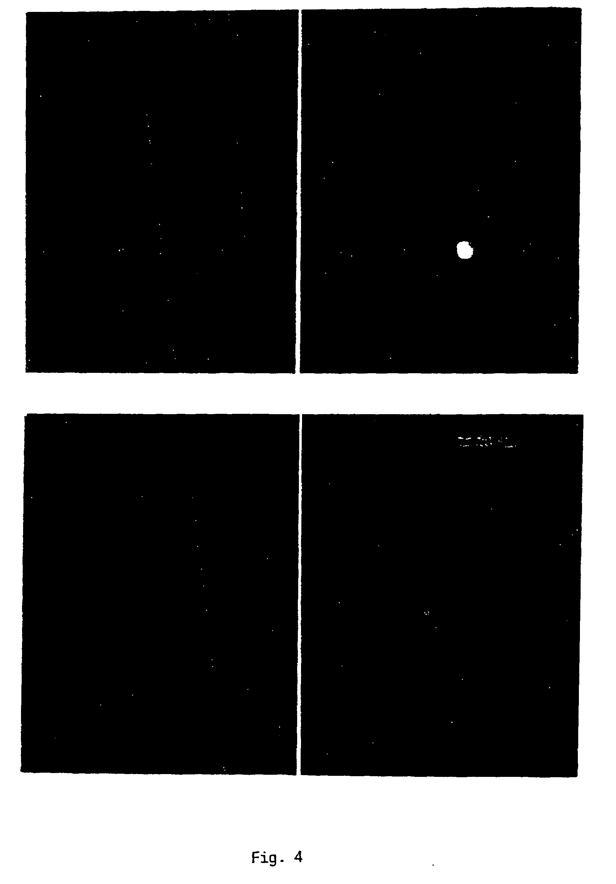

Etwa 12 Stunden nach Transfektion haben 20-60% eine schwache Grünfluoreszenz im Zellkern (nicht gezeigt). Dies verweist auf die zunächst moderate Expression des Fusionsproteins. Morphologisch sind keine Besonderheiten zu erkennen. Ab etwa 24 Stunden nach Transfektion des Vektorkonstrukts treten in einzelnen Zellen Verdichtungen des Fusionsproteins auf (Fig. 3 links). Diese Verdichtungen sind auch im Phasenkontrastbild (Fig. 4) zu beobachten. Im weiteren zeitlichen Verlauf werden diese Verdichtungen stärker (Fig. 3 von links nach rechts) und das Phasenkontrastbild (Fig. 4) entspricht dem typischen Bild einer Zelle in Apoptose.

Nicht alle Zellen, die gleichzeitig transfiziert wurden, zeigen auch gleichzeitig die im Bild zu sehende übersteigerte Expressionsrate des Fusionsproteins. Zellen wie in Fig. 3 links werden auch noch nach 48-72 Stunden beobachtet, wohingegen andere Zellen schon den Endpunkt (Fig. 3 rechts) erreicht haben. Dies zeigt, daß die Zellen einer Kultur "gestaffelt" in den Apoptose-Prozeß eintreten. Bei ausreichend hoher Transfektionsrate sind letzlich alle Zellen einer Kultur, auch solche, die nicht transfiziert wurden, abgetötet. Dieser Zeitpunkt ist abhängig von der initialen Transfektionsrate. Durch die Apoptose der transfizierten Zellen werden Faktoren abgegeben, die für nicht-transfizierte Zellen in der Kultur schädlich sind und schließlich zu Abtötung auch dieser nicht-transfizierten Zellen führen (sog. "Bystander-Effekt").

Es soll angemerkt werden, daß GFP (grün fluoreszierendes Protein von Aequorea Victoria) nur zur Unterscheidung transfizierter und nicht-transfizierter Zellen verwendet wurde bzw. zur Sichtbarmachung der Überexpression. GFP-Expression allein hat keinerlei Effekt auf die Zellmorphologie bzw. auf die Überlebensfähigkeit von Zellen. GFP-Fusionsproteine haben grundsätzlich die funktionellen Eigenschaften (und auch die intrazellulären Verteilungen) wie das funktionelle Genprodukt. Die in den Figuren gezeigten apoptotischen Prozesse beruhen deshalb auf einer C1D-Funktion. Die gezeigte Morphologie (und der Zellzahlverlust) wurde in Kontrollexperimenten auch durch Konstrukte bewirkt, die nur die C1D-Sequenz enthielten (z.B. durch die oben beschriebenen pcDNA 3-C1D-Expressionskontrukte).

## Patentansprüche

1. C1D-Gen zur Verwendung als Arzneimittel zur Auslösung von Apoptose in Zellen durch Überexpression des C1D-Gens.

2. C1D-Gen nach Anspruch 2, wobei die Zellen Tumorzellen sind.

3. C 1D-Gen nach Anspruch 1 oder 2, wobei das C 1D-Genprodukt die Aminosäuresequenz von Fig. 1 bzw. 2 oder eine hiervon durch eine oder mehrere Aminosäuren unterschiedliche Aminosäurensequenz aufweist, wobei die DNA-Sequenz der letzteren Aminosäuresequenz mit der DNA von Fig.1 bzw. 2 unter stringenten Bedingungen bei einer Hybridisierungstemperatur von ca. 65°C hybridisiert.

4. C1D-Gen nach einem der Ansprüche 1 bis 3, wobei das C1D-Gen enthalten ist in einem Expressionsvektor zur Transfektion der Zellen als
(a) DNA von Fig. 1 bzw. 2 oder eine hiervon durch ein oder mehrere Basenpaare unterschiedliche DNA, wobei letztere mit der DNA von Fig. 1 bzw. 2 unter stringenten Bedingungen bei einer Hybridisierungstemperatur von ca. 65°C hybridisiert, oder
(b) eine mit der DNA von (a) über den degenerierten genetischen Code verwandte DNA.

5. C1D-Gen nach einem der Ansprüche 1 bis 3, wobei das C1D-Gen endogen in den Zellen enthalten ist.

6. C1D-Gen nach Anspruch 5, wobei der Promotor des endogenen C1D-Gens durch extrazelluläre Faktoren stimulierbar ist.

## Claims

1. CID gene for use as a medicament to induce apoptosis in cells by overexpression of the CID gene.

2. CID gene according to claim 1, wherein the cells are tumor cells.

3. CID gene according to claim 1 or 2. wherein the CID gene product includes the amino acid sequence of figure 1 and/or 2 or an amino acid sequence differing therefrom by one or several amino acids, the DNA sequence of the latter amino acid sequence hybridizing with the DNA of figure 1 and/or 2 under stringent conditions at a hybridization temperature of about 65°C.

4. CID gene according to any of claims 1 to 3, wherein the CID gene is contained in an expression vector for the transfection of the cells as
(a) DNA of figure 1 and/or 2 or a DNA differing therefrom by one or several base pairs, the latter hybridizing with the DNA of figure 1 and/or 2 under stringent conditions at a hybridization temperature of about 65°C, or
(b) a DNA related to the DNA of (a) via the degenerated genetic code.

5. CID gene according to any of claims 1 to 3, wherein the CID gene is endogenously contained in the cells.

6. CID gene according to claim 5, wherein the promoter of the endogenous CID gene can be stimulated by extracellular factors.

## Revendications

1. Gène C1D destiné à être utilisé comme médicament pour déclencher l'apoptose dans des cellules par surexpression du gène C1D.

2. Gène C1D suivant la revendication 2, pour lequel les cellules sont des cellules tumorales.

3. Gène C1D suivant la revendication 1 ou 2, pour lequel le produit génique C1D présente la séquence d'aminoacides de la figure 1 ou 2 ou une séquence d'aminoacides qui en diffère d'un ou plusieurs aminoacides, la séquence d'ADN de la dernière séquence d'aminoacides s'hybridant avec l'ADN de la figure 1 ou 2 dans des conditions stringentes à une température d'hybridation d'environ 65°C.

4. Gène C1D suivant l'une des revendications 1 à 3, ce gène C1D étant contenu dans un vecteur d'expression pour la transfection des cellules comme
(a) ADN de la figure 1 ou 2 ou un ADN qui en diffère d'une ou plusieurs paires de bases, ce dernier ADN s'hybridant avec l'ADN de la figure 1 ou 2 dans des conditions stringentes à une température d'hybridation d'environ 65°C, ou
(b) un ADN apparenté à l'ADN de (a) par l'intermédiaire d'un code génétique dégénéré.

5. Gène C1D suivant l'une des revendications 1 à 3, ce gène C1D étant contenu de manière endogène dans les cellules.

6. Gène C1D suivant la revendication 5, le promoteur du gène C1D endogène pouvant être stimulé par des facteurs extracellulaires.
